# EUROPEAN PATENT APPLICATION

(11) **EP 4 360 547 A1**
(43) Date of publication of application: **01.05.2024**
(21) Application number: 22204074.3
(22) Date of filing: 27.10.2022
(51) Int. Cl.: A61B 5/055, A61B 5/11, A61B 5/00

(54) **PATIENT POSE DETECTION APPARATUS**

(71) Applicant: Koninklijke Philips N.V., 5656 AG Eindhoven (NL)
(72) Inventor: SOMMER, Karsten, Eindhoven (NL); KRUEGER, Sascha, Eindhoven (NL)
(74) Representative: Philips Intellectual Property & Standards

(57) **Abstract**

The present invention relates to a patient pose detection apparatus (10), comprising:
- at least one millimetre wave and/or terahertz sensor (20); and
- a processing unit (30);
wherein the at least one millimetre wave and/or terahertz sensor is configured to be located in the vicinity of a patient on a patient support of a medical imaging unit;
wherein the at least one millimetre wave and/or terahertz sensor is configured to acquire millimetre wave and/or terahertz image data of the patient on the patient support of the medical imaging unit prior to medical image data of the patient being acquired by the medical imaging unit;
wherein the at least one millimetre wave and/or terahertz sensor is configured to provide the millimetre wave and/or terahertz image data of the patient on the patient support of the medical imaging unit to the processing unit; and
wherein the processing unit is configured to determine a pose of the patient on the patient support of the medical imaging unit, wherein the determination of the pose of the patient on the patient support comprises utilization of the millimetre wave and/or terahertz image data of the patient on the patient support of the medical imaging unit.

## Description

### FIELD OF THE INVENTION

The present invention relates to a patient pose detection apparatus, a patient pose detection system, a patient pose detection method, a computer program element, and a computer readable medium.

### BACKGROUND OF THE INVENTION

The preparation of the patient for a medical imaging scan, exam or examination is a time-consuming and error-prone task. To simplify and accelerate the clinical workflow, automation of this part of the exam using optical sensors (cameras) is a viable option. One of the key requirements for such a system is highly accurate localization of anatomical keypoints or landmarks of the patient, which is used for example to automatically determine the scan position, the pose of the patient (feet-first/head-first, supine/prone, etc.), or for safety features such as collision detection.

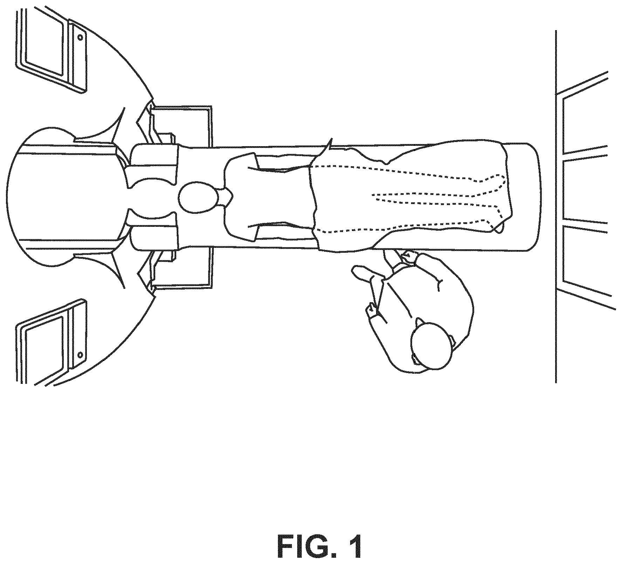

When relying on optical sensors, occlusion of the patient can complicate anatomical keypoint or landmark localization. Fig. 1 shows an example of such a case, where a blanket has been placed onto the patient, making accurate localization of lower extremity keypoints or landmarks impossible.

There is a need to address these problems.

### SUMMARY OF THE INVENTION

It would be advantageous to have an improved technique helping acquire the pose of a patient prior to medical image scans. The object of the present invention is solved with the subject matter of the independent claims, wherein further embodiments are incorporated in the dependent claims.

In a first aspect, there is provided a patient pose detection apparatus, comprising:
- at least one millimetre wave and/or terahertz sensor; and
- a processing unit.

The at least one millimetre wave and/or terahertz sensor is configured to be located in the vicinity of a patient on a patient support of a medical imaging unit. The at least one millimetre wave and/or terahertz sensor is configured to acquire millimetre wave and/or terahertz image data of the patient on the patient support of the medical imaging unit prior to medical image data of the patient being acquired by the medical imaging unit. The at least one millimetre wave and/or terahertz sensor is configured to provide the millimetre wave and/or terahertz image data of the patient on the patient support of the medical imaging unit to the processing unit. The processing unit is configured to determine a pose of the patient on the patient support of the medical imaging unit. The determination of the pose of the patient on the patient support comprises utilization of the millimetre wave and/or terahertz image data of the patient on the patient support of the medical imaging unit.

In this manner, mm wave radiation and/or terahertz radiation that can propagate through various materials enables a pose of a patient on a patient support such as a bed to be determined even when the patient is occluded such as being partially covered with a blanket. This ensures that the patient can be correctly positioned for a medical imaging scan or examination within a medical imaging unit such as a CT scanner, MRI scanner, PET scanner etc, prior to being placed within the medical imaging unit.

It is to be noted that just mm wave sensing 30-300GHz can be utilized or just terahertz sensing 300-3000GHz can be utilized, as both modalities enable images through occlusions to be acquired. However, by using both a mm Wave sensor and a Terahertz sensor the bandwidth of images can be effectively increased.

In an example, the at least one millimetre wave and/or terahertz sensor is configured to move to a plurality of positions to acquire the millimetre wave image data of the patient on the patient support of the medical imaging unit.

In this way, by moving one or more mm wave and/or terahertz sensors a higher resolution mm wave image of the patient can be obtained, improving the ability to accurately determine the patient's pose.

In an example, the at least one millimetre wave and/or terahertz sensor comprises a plurality of millimetre wave and/or terahertz sensors spaced apart from one another.

By using a number of mm wave and/or terahertz sensors viewing the patient from different locations and therefore from different angles, a higher resolution mm wave and/or terahertz image of the patient can be obtained, improving the ability to accurately determine the patient's pose.

In an example, the apparatus comprises a visible or infrared sensor. The visible or infrared sensor is configured to be located in the vicinity of the patient on the patient support of the medical imaging unit. The visible or infrared sensor is configured to acquire visible or infrared image data of the patient on the patient support of the medical imaging unit prior to medical image data of the patient being acquired by the medical imaging unit. The visible or infrared sensor is configured to provide the visible or infrared image data of the patient on the patient support of the medical imaging unit to the processing unit. The determination of the pose of the patient on the patient support can comprise utilization of the visible or infrared image data of the patient on the patient support of the medical imaging unit.

In this manner, high resolution visible or infrared imagery is used to augment the mm wave and/or terahertz imagery enabling the pose of the patient to be better determined.

In an example, the millimetre wave and/or terahertz image data of the patient on the patient support of the medical imaging unit comprises a first millimetre wave and/or terahertz image of the patient on the patient support of the medical imaging unit acquired at a first time frame and a second millimetre wave and/or terahertz image of the patient on the patient support of the medical imaging unit acquired at a second time frame after the first time frame. The visible or infrared image data of the patient on the patient support of the medical imaging unit comprises a first visible or infrared image of the patient on the patient support of the medical imaging unit acquired contemporaneously with the first millimetre wave and/or terahertz image of the patient on the patient support of the medical imaging unit.

Thus, a first millimetre wave image and a second millimetre wave image can be acquired, or a first terahertz image and a second terahertz image can be acquired. When both a millimetre wave sensor and a terahertz sensor are used, then they acquire contemporaneous images that can be in effect fused to form a first mm wave/terahertz image and at a later acquisition time again fused to form a second mm wave/terahertz image.

In this manner, a first mm wave and/or terahertz image and associated visible image can be acquired prior to a patient being partially occluded, for example having a blanket placed over a part of their body. The non-occluded visible or infrared image and the associated mm wave and/or terahertz image can then be used together to provide information on how a mm wave and/or terahertz image can be better interpreted with respect to a pose of the patient. Then the patient can be at least partially occluded, for example having a blanket placed over a part of their body. A subsequent mm wave and/or terahertz image is acquired that can see through the occlusion (see through the blanket). The information derived from the non-occluded visible or infrared image and the associated mm wave and/or terahertz image can then be used to better interpret the mm wave and/or terahertz image of the occluded patient enabling a pose of the patient to be accurately determined.

In an example, the processing unit is configured to determine locations of a plurality of landmarks of the patient at the first time frame. The determination of the locations of the plurality of landmarks of the patient at the first time frame comprises utilization of the first millimetre wave and/or terahertz image of the patient on the patient support of the medical imaging unit and the first visible or infrared image of the patient on the patient support of the medical imaging unit. The processing unit is configured to determine locations of the plurality of landmarks of the patient at the second time frame. The determination of the locations of the plurality of landmarks of the patient at the second time frame comprises utilization of the determined locations of the plurality of landmarks of the patient at the first time frame and the second millimetre and/or terahertz wave image of the patient on the patient support of the medical imaging unit. The determination of the pose of the patient on the patient support can comprise utilization of the determined locations of the plurality of landmarks of the patient at the second time frame.

In other words, the first mm wave and/or terahertz image and the associated visible or infrared image are used to determine locations of landmark features such as ankles, knees, hips, writs, elbows, shoulders and head of a patient, where the high resolution visible/infrared image helps locate the landmarks within the mm wave and/or terahertz image. Then in effect this combined mm Wave(Terahertz)/visible or infrared image and its landmarks can be used to aid location of the same landmarks of the patient in a subsequent mm wave image of the patient, where the patient is now partially occluded. This facilities the accurate determination of the pose of the patient when they are partially occluded.

In an example, the visible or infrared image data of the patient on the patient support of the medical imaging unit comprises a second visible or infrared image of the patient on the patient support of the medical imaging unit acquired contemporaneously with the second millimetre wave and/or terahertz image of the patient on the patient support of the medical imaging unit.

In an example, the determination of the locations of the plurality of landmarks of the patient at the second time frame comprises utilization of the second visible or infrared image of the patient on the patient support of the medical imaging unit.

In this manner, visible/infrared and mm wave and/or terahertz image data are used in combination both before and after a patient is partially occluded (with for example a blanket covering a part of their body) to enable the pose of the patient to be determined when they are partially occluded.

In an example, the determination of the pose of the patient on the patient support comprises utilization of a trained neural network.

In an example, the trained neural network has been trained on the basis of a plurality of image pairs of one or more reference persons. The plurality of image pairs comprises millimetre wave and/or terahertz images of the one or more reference persons and associated visible or infrared images of the one or more persons acquired contemporaneously with the millimetre wave and/or terahertz images of the one or more reference persons.

In an example, the plurality of image pairs of one or more reference persons comprises image data of the one or more reference persons with different degrees of body occlusion with respect to visible or infrared image data.

Thus, the neural network is trained using both mm wave and visible/infrared images of persons who are not occluded in both images of a first image set and who are occluded to varying degrees in both images of further image sets. This then enables the neural network to receive a mm wave and visible/infrared image pair for a non-occluded patient and receive a later mm wave and visible/infrared image pair for an occluded patient from which the pose of the occluded patient can be accurately determined.

In a second aspect, there is provided a patient pose detection system, comprising:
- a medical imaging unit;
- at least one millimetre wave and/or terahertz sensor; and
- a processing unit.

The at least one-millimetre wave and/or terahertz sensor is located in the vicinity of a patient on a patient support of the medical imaging unit. The at least one millimetre wave and/or terahertz sensor is configured to acquire millimetre wave and/or terahertz image data of the patient on the patient support of the medical imaging unit prior to medical image data of the patient being acquired by the medical imaging unit. The at least one millimetre wave and/or terahertz sensor is configured to provide the millimetre wave and/or terahertz image data of the patient on the patient support of the medical imaging unit to the processing unit. The processing unit is configured to determine a pose of the patient on the patient support of the medical imaging unit. The determination of the pose of the patient on the patient support comprises utilization of the millimetre wave and/or terahertz image data of the patient on the patient support of the medical imaging unit.

In a third aspect, there is provided a patient pose detection method, comprising: acquiring by at least one millimetre wave and/or terahertz sensor located in the vicinity of a patient on a patient support of a medical imaging unit millimetre wave and/or terahertz image data of the patient on the patient support of the medical imaging unit prior to medical image data of the patient being acquired by the medical imaging unit;
providing by the at least one millimetre wave and/or terahertz sensor the millimetre wave and/or terahertz image data of the patient on the patient support of the medical imaging unit to a processing unit; and
determining by the processing unit a pose of the patient on the patient support of the medical imaging unit, wherein the determining the pose of the patient on the patient support comprises utilizing the millimetre wave and/or terahertz image data of the patient on the patient support of the medical imaging unit.

In an aspect, there is provided a computer program element for controlling an apparatus according to the first aspect which when executed by a processor is configured to carry out the method of the third aspect.

In an aspect, there is provided a computer program element for controlling a system according to the second aspect which when executed by a processor is configured to carry out the method of the third aspect.

Thus, according to aspects, there is provided computer program elements controlling one or more of the apparatuses/systems as previously described which, if the computer program element is executed by a processor, is adapted to perform the method as previously described.

According to another aspect, there is provided computer readable media having stored the computer elements as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawing:
Fig. 1 shows a patient preparation scene prior to imaging with a medical imaging unit where the patient is subject to partial occlusion due to a blanket, making localization of lower extremities extremely difficult;
Fig. 2 shows an example of a patient pose detection apparatus;
Fig. 3 shows an example of a patient pose detection system;
Fig. 4 shows a patient pose detection method; and
Fig. 5 shows a workflow of a detailed embodiment of the patient pose detection apparatus, system and method.

### DETAILED DESCRIPTION OF EMBODIMENTS

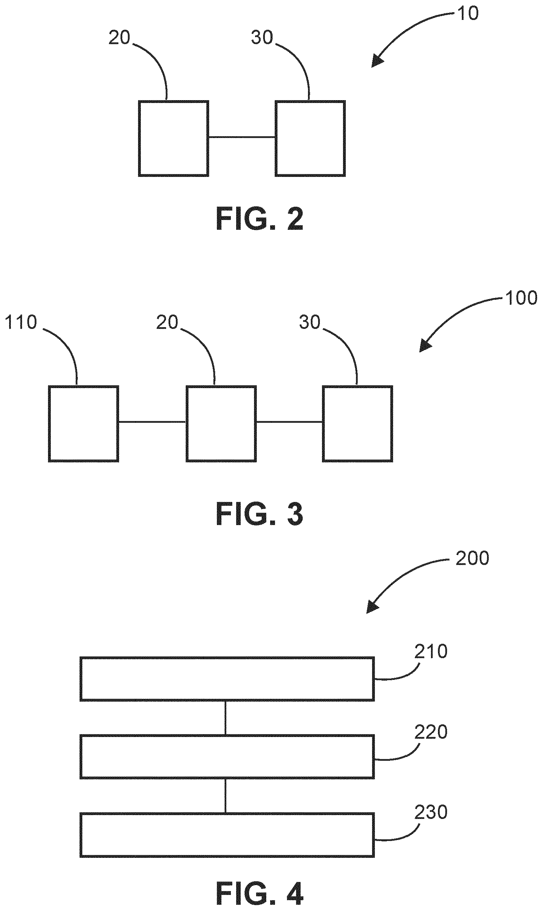

Fig. 2 shows an example of a patient pose detection apparatus 10 comprising at least one millimetre wave and/or terahertz sensor 20, and a processing unit 30. The at least one millimetre wave and/or terahertz sensor is configured to be located in the vicinity of a patient on a patient support of a medical imaging unit. The at least one millimetre wave and/or terahertz sensor is configured to acquire millimetre wave and/or terahertz image data of the patient on the patient support of the medical imaging unit prior to medical image data of the patient being acquired by the medical imaging unit. The at least one millimetre wave and/or terahertz sensor is configured to provide the millimetre wave and/or terahertz image data of the patient on the patient support of the medical imaging unit to the processing unit. The processing unit is configured to determine a pose of the patient on the patient support of the medical imaging unit. The determination of the pose of the patient on the patient support comprises utilization of the millimetre wave and/or terahertz image data of the patient on the patient support of the medical imaging unit.

In this manner, mm wave radiation and/or terahertz radiation that can propagate through various materials enables a pose of a patient on a patient support such as a bed to be determined even when the patient is occluded such as being partially covered with a blanket. This ensures that the patient can be correctly positioned for a medical imaging scan or examination within a medical imaging unit such as a CT scanner, MRI scanner, PET scanner etc, prior to being placed within the medical imaging unit.

It is to be noted that just mm wave sensing 30-300GHz can be utilized or just terahertz sensing 300-3000GHz can be utilized, as both modalities enable images through occlusions to be acquired. However, by using a mm Wave sensor and a Terahertz sensor the bandwidth of images can be effectively increased.

In an example, the processing unit is configured to control the at least one millimetre wave and/or terahertz sensor to acquire the millimetre wave and/or terahertz image data of the patient on the patient support of the medical imaging unit prior to medical image data of the patient being acquired by the medical imaging unit.

According to an example, the at least one millimetre wave and/or terahertz sensor is configured to move to a plurality of positions to acquire the millimetre wave image data of the patient on the patient support of the medical imaging unit.

In this way, by moving one or more mm wave and/or terahertz sensors a higher resolution mm wave image of the patient can be obtained, improving the ability to accurately determine the patient's pose.

According to an example, the at least one millimetre wave and/or terahertz sensor comprises a plurality of millimetre wave and/or terahertz sensors spaced apart from one another.

By using a number of mm wave and/or terahertz sensors viewing the patient from different locations and therefore from different angles, a higher resolution mm wave and/or terahertz image of the patient can be obtained, improving the ability to accurately determine the patient's pose.

According to an example, the apparatus comprises a visible or infrared sensor. The visible or infrared sensor is configured to be located in the vicinity of the patient on the patient support of the medical imaging unit. The visible or infrared sensor is configured to acquire visible or infrared image data of the patient on the patient support of the medical imaging unit prior to medical image data of the patient being acquired by the medical imaging unit. The visible or infrared sensor is configured to provide the visible or infrared image data of the patient on the patient support of the medical imaging unit to the processing unit. The determination of the pose of the patient on the patient support can comprise utilization of the visible or infrared image data of the patient on the patient support of the medical imaging unit.

In this manner, high resolution visible or infrared imagery is used to augment the mm wave and/or terahertz imagery enabling the pose of the patient to be better determined.

In an example, the processing unit is configured to control the visible or infrared sensor to acquire the visible or infrared image data of the patient on the patient support of the medical imaging unit prior to medical image data of the patient being acquired by the medical imaging unit.

According to an example, the millimetre wave and/or terahertz image data of the patient on the patient support of the medical imaging unit comprises a first millimetre wave and/or terahertz image of the patient on the patient support of the medical imaging unit acquired at a first time frame and a second millimetre wave and/or terahertz image of the patient on the patient support of the medical imaging unit acquired at a second time frame after the first time frame. The visible or infrared image data of the patient on the patient support of the medical imaging unit comprises a first visible or infrared image of the patient on the patient support of the medical imaging unit acquired contemporaneously with the first millimetre wave and/or terahertz image of the patient on the patient support of the medical imaging unit.

Thus, a first millimetre wave image and a second millimetre wave image can be acquired, or a first terahertz image and a second terahertz image can be acquired. When both a millimetre wave sensor and a terahertz sensor are used, then they acquire contemporaneous images that can be in effect fused to form a first mm wave/terahertz image and at a later acquisition time again fused to form a second mm wave/terahertz image.

In this manner, a first mm wave and/or terahertz image and associated visible image can be acquired prior to a patient being partially occluded, for example having a blanket placed over a part of their body. The non-occluded visible or infrared image and the associated mm wave and/or terahertz image can then be used together to provide information on how a mm wave and/or terahertz image can be better interpreted with respect to a pose of the patient. Then the patient can be at least partially occluded, for example having a blanket placed over a part of their body. A subsequent mm wave and/or terahertz image is acquired that can see through the occlusion (see through the blanket). The information derived from the non-occluded visible or infrared image and the associated mm wave and/or terahertz image can then be used to better interpret the mm wave and/or terahertz image of the occluded patient enabling a pose of the patient to be accurately determined.

According to an example, the processing unit is configured to determine locations of a plurality of landmarks of the patient at the first time frame. The determination of the locations of the plurality of landmarks of the patient at the first time frame comprises utilization of the first millimetre wave and/or terahertz image of the patient on the patient support of the medical imaging unit and the first visible or infrared image of the patient on the patient support of the medical imaging unit, and the processing unit is configured to determine locations of the plurality of landmarks of the patient at the second time frame. The determination of the locations of the plurality of landmarks of the patient at the second time frame comprises utilization of the determined locations of the plurality of landmarks of the patient at the first time frame and the second millimetre and/or terahertz wave image of the patient on the patient support of the medical imaging unit. The determination of the pose of the patient on the patient support can comprise utilization of the determined locations of the plurality of landmarks of the patient at the second time frame.

In other words, the first mm wave and/or terahertz image and the associated visible or infrared image are used to determine locations of landmark features such as ankles, knees, hips, writs, elbows, shoulders and head of a patient, where the high resolution visible/infrared image helps locate the landmarks within the mm wave and/or terahertz image. Then in effect this combined mm Wave(Terahertz)/visible or infrared image and its landmarks can be used to aid location of the same landmarks of the patient in a subsequent mm wave image of the patient, where the patient is now partially occluded. This facilities the accurate determination of the pose of the patient when they are partially occluded.

According to an example, the visible or infrared image data of the patient on the patient support of the medical imaging unit comprises a second visible or infrared image of the patient on the patient support of the medical imaging unit acquired contemporaneously with the second millimetre wave and/or terahertz image of the patient on the patient support of the medical imaging unit.

According to an example, the determination of the locations of the plurality of landmarks of the patient at the second time frame comprises utilization of the second visible or infrared image of the patient on the patient support of the medical imaging unit.

In this manner, visible/infrared and mm wave and/or terahertz image data are used in combination both before and after a patient is partially occluded (with for example a blanket covering a part of their body) to enable the pose of the patient to be determined when they are partially occluded.

According to an example, the determination of the pose of the patient on the patient support comprises utilization of a trained neural network.

According to an example, the trained neural network has been trained on the basis of a plurality of image pairs of one or more reference persons. The plurality of image pairs comprises millimetre wave and/or terahertz images of the one or more reference persons and associated visible or infrared images of the one or more persons acquired contemporaneously with the millimetre wave and/or terahertz images of the one or more reference persons.

In an example, the plurality of image pairs are reference images of persons on a patient support. Thus, it is possible to train the neural network with images of persons that are not on a patient support, and that indeed can be with completely different scenes, and the neural network will perform well for patient support scenes. However, the reference image pairs can include a number of image pairs of persons on patient supports.

According to an example, the plurality of image pairs of one or more reference persons comprises image data of the one or more reference persons with different degrees of body occlusion with respect to visible or infrared image data.

Thus, the neural network is trained using both mm wave and visible/infrared images of persons who are not occluded in both images of a first image set and who are occluded to varying degrees in both images of further image sets. This then enables the neural network to receive a mm wave and visible/infrared image pair for a non-occluded patient and receive a later mm wave and visible/infrared image pair for an occluded patient from which the pose of the occluded patient can be accurately determined.

Fig. 3 shows an example of a patient pose detection system 100, comprising a medical imaging unit 110, at least one millimetre wave and/or terahertz sensor 20, and a processing unit 30. The at least one-millimetre wave and/or terahertz sensor is located in the vicinity of a patient on a patient support of the medical imaging unit. The at least one millimetre wave and/or terahertz sensor is configured to acquire millimetre wave and/or terahertz image data of the patient on the patient support of the medical imaging unit prior to medical image data of the patient being acquired by the medical imaging unit. The at least one millimetre wave and/or terahertz sensor is configured to provide the millimetre wave and/or terahertz image data of the patient on the patient support of the medical imaging unit to the processing unit. The processing unit is configured to determine a pose of the patient on the patient support of the medical imaging unit. The determination of the pose of the patient on the patient support comprises utilization of the millimetre wave and/or terahertz image data of the patient on the patient support of the medical imaging unit.

In an example, the processing unit is configured to control the at least one millimetre wave and/or terahertz sensor to acquire the millimetre wave and/or terahertz image data of the patient on the patient support of the medical imaging unit prior to medical image data of the patient being acquired by the medical imaging unit.

In an example, the at least one millimetre wave and/or terahertz sensor is configured to move to a plurality of positions to acquire the millimetre wave and/or terahertz image data of the patient on the patient support of the medical imaging unit.

In an example, the at least one millimetre wave and/or terahertz sensor comprises a plurality of millimetre wave and/or terahertz sensors spaced apart from one another.

In an example, the system comprises a visible or infrared sensor. The visible or infrared sensor is located in the vicinity of the patient on the patient support of the medical imaging unit. The visible or infrared sensor is configured to acquire visible or infrared image data of the patient on the patient support of the medical imaging unit prior to medical image data of the patient being acquired by the medical imaging unit. The visible or infrared sensor is configured to provide the visible or infrared image data of the patient on the patient support of the medical imaging unit to the processing unit. The determination of the pose of the patient on the patient support can comprise utilization of the visible or infrared image data of the patient on the patient support of the medical imaging unit.

In an example, the processing unit is configured to control the visible or infrared sensor to acquire the visible or infrared image data of the patient on the patient support of the medical imaging unit prior to medical image data of the patient being acquired by the medical imaging unit.

In an example, the millimetre wave and/or terahertz image data of the patient on the patient support of the medical imaging unit comprises a first millimetre wave and/or terahertz image of the patient on the patient support of the medical imaging unit acquired at a first time frame and a second millimetre wave and/or terahertz image of the patient on the patient support of the medical imaging unit acquired at a second time frame after the first time frame. The visible or infrared image data of the patient on the patient support of the medical imaging unit comprises a first visible or infrared image of the patient on the patient support of the medical imaging unit acquired contemporaneously with the first millimetre wave and/or terahertz image of the patient on the patient support of the medical imaging unit.

In an example, the processing unit is configured to determine locations of a plurality of landmarks of the patient at the first time frame. The determination of the locations of the plurality of landmarks of the patient at the first time frame comprises utilization of the first millimetre wave and/or terahertz image of the patient on the patient support of the medical imaging unit and the first visible or infrared image of the patient on the patient support of the medical imaging unit. The processing unit is configured to determine locations of the plurality of landmarks of the patient at the second time frame, wherein the determination of the locations of the plurality of landmarks of the patient at the second time frame comprises utilization of the determined locations of the plurality of landmarks of the patient at the first time frame and the second millimetre wave and/or terahertz image of the patient on the patient support of the medical imaging unit. The determination of the pose of the patient on the patient support comprises utilization of the determined locations of the plurality of landmarks of the patient at the second time frame.

In an example, the visible or infrared image data of the patient on the patient support of the medical imaging unit comprises a second visible or infrared image of the patient on the patient support of the medical imaging unit acquired contemporaneously with the second millimetre wave and/or terahertz image of the patient on the patient support of the medical imaging unit.

In an example, the determination of the locations of the plurality of landmarks of the patient at the second time frame comprises utilization of the second visible or infrared image of the patient on the patient support of the medical imaging unit.

In an example, the determination of the pose of the patient on the patient support comprises utilization of a trained neural network.

In an example, the trained neural network has been trained on the basis of a plurality of image pairs of one or more reference persons. The plurality of image pairs comprises millimetre wave and/or terahertz images of the one or more reference persons and associated visible or infrared images of the one or more reference persons acquired contemporaneously with the millimetre wave and/or terahertz images of the one or more reference persons.

In an example, the plurality of image pairs of one or more reference persons comprises image data of the one or more reference persons with different degrees of body occlusion with respect to visible or infrared image data.

Fig. 4 shows a patient pose detection method 200, comprising:
acquiring 210 by at least one millimetre wave and/or terahertz sensor located in the vicinity of a patient on a patient support of a medical imaging unit millimetre wave and/or terahertz image data of the patient on the patient support of the medical imaging unit prior to medical image data of the patient being acquired by the medical imaging unit;
providing 220 by the at least one millimetre wave and/or terahertz sensor the millimetre wave and/or terahertz image data of the patient on the patient support of the medical imaging unit to a processing unit; and
determining 230 by the processing unit a pose of the patient on the patient support of the medical imaging unit, wherein the determining the pose of the patient on the patient support comprises utilizing the millimetre wave and/or terahertz image data of the patient on the patient support of the medical imaging unit.

In an example, the method comprises controlling by the processing unit the at least one millimetre wave and/or terahertz sensor to acquire the millimetre wave and/or terahertz image data of the patient on the patient support of the medical imaging unit prior to medical image data of the patient being acquired by the medical imaging unit.

In an example, the method comprises moving the at least one millimetre wave and/or terahertz sensor to a plurality of positions to acquire the millimetre wave and/or terahertz image data of the patient on the patient support of the medical imaging unit.

In an example, the at least one millimetre wave and/or terahertz sensor comprises a plurality of millimetre wave and/or terahertz sensors spaced apart from one another.

In an example, the system comprises a visible or infrared sensor, wherein the visible or infrared sensor located in the vicinity of the patient on the patient support of the medical imaging unit. The method comprises acquiring by the visible or infrared sensor visible or infrared image data of the patient on the patient support of the medical imaging unit prior to medical image data of the patient being acquired by the medical imaging unit. The method comprises providing by the visible or infrared sensor the visible or infrared image data of the patient on the patient support of the medical imaging unit to the processing unit. The determining the pose of the patient on the patient support can comprise utilizing the visible or infrared image data of the patient on the patient support of the medical imaging unit.

In an example, the method comprises controlling by the processing unit the visible or infrared sensor to acquire the visible or infrared image data of the patient on the patient support of the medical imaging unit prior to medical image data of the patient being acquired by the medical imaging unit.

In an example, the millimetre wave and/or terahertz image data of the patient on the patient support of the medical imaging unit comprises a first millimetre wave and/or terahertz image of the patient on the patient support of the medical imaging unit acquired at a first time frame and a second millimetre wave and/or terahertz image of the patient on the patient support of the medical imaging unit acquired at a second time frame after the first time frame. The visible or infrared image data of the patient on the patient support of the medical imaging unit comprises a first visible or infrared image of the patient on the patient support of the medical imaging unit acquired contemporaneously with the first millimetre wave and/or terahertz image of the patient on the patient support of the medical imaging unit.

In an example, the method comprises determining by the processing unit locations of a plurality of landmarks of the patient at the first time frame. The determining the locations of the plurality of landmarks of the patient at the first time frame comprises utilizing the first millimetre wave and/or terahertz image of the patient on the patient support of the medical imaging unit and the first visible or infrared image of the patient on the patient support of the medical imaging unit. The method comprises determining by the processing unit locations of the plurality of landmarks of the patient at the second time frame. The determining the locations of the plurality of landmarks of the patient at the second time frame comprises utilizing the determined locations of the plurality of landmarks of the patient at the first time frame and the second millimetre wave and/or terahertz image of the patient on the patient support of the medical imaging unit. The determining the pose of the patient on the patient support can comprise utilizing the determined locations of the plurality of landmarks of the patient at the second time frame.

In an example, the visible or infrared image data of the patient on the patient support of the medical imaging unit comprises a second visible or infrared image of the patient on the patient support of the medical imaging unit acquired contemporaneously with the second millimetre wave and/or terahertz image of the patient on the patient support of the medical imaging unit.

In an example, the determining the locations of the plurality of landmarks of the patient at the second time frame comprises utilizing the second visible or infrared image of the patient on the patient support of the medical imaging unit.

In an example, the determining the pose of the patient on the patient support comprises utilizing a trained neural network.

In an example, the trained neural network has been trained on the basis of a plurality of image pairs of one or more reference persons. The plurality of image pairs comprises millimetre wave and/or terahertz images of the one or more reference persons and associated visible or infrared images of the one or more reference persons acquired contemporaneously with the millimetre wave and/or terahertz images of the one or more reference persons.

In an example, the plurality of image pairs of one or more reference persons comprises image data of the one or more reference persons with different degrees of body occlusion with respect to visible or infrared image data.

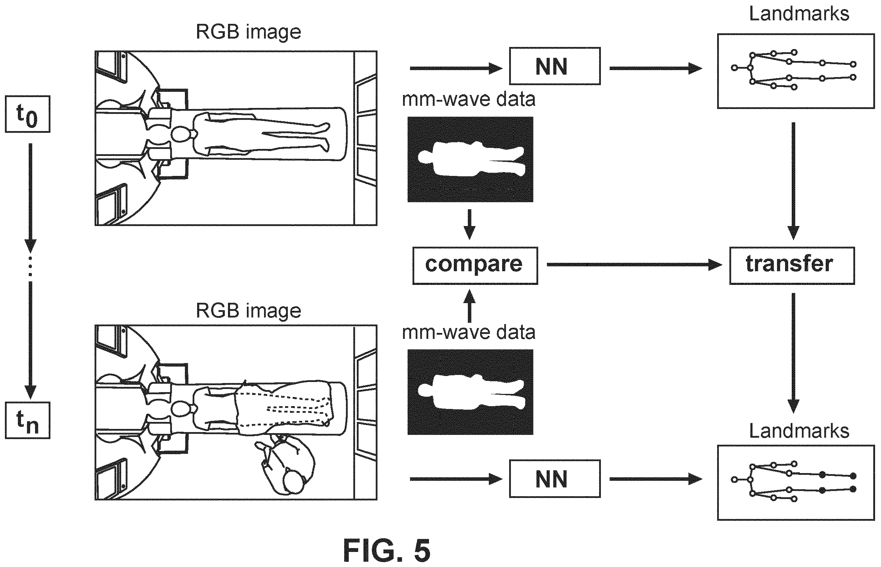

The patient pose detection apparatus, system and method are now described in further detail, where reference is made to Fig. 5, where the discussion centers on millimetre wave radiation but has applicability to terahertz radiation as well.

It was realised by the inventors that millimetre wave radiation, sometimes referred to as millimetre RF wave radiation, could be utilized in a completely new way to enable the pose of a patient being prepared for a medical imaging scan or exam/examination to be determined. Millimetre wave sensors or scanners have been developed to detect concealed objects, for example for airport security. See for example: D. McMakin etc al "New Improvements to Millimeter -Wave Body Scanners", Proceedings of 3DBODY.TECH 2017, 8th International Conference and Exhibition on 3D Body Scanning and Processing Technologies, Montreal, Canada, 11-12 Oct. 2017, pages 263-271; D Li et al "Multi-Person Action Recognition in Microwave Sensors", Oral Session HI: Emerging Multimedia Applications, MM '20, October 12-16, 2020, Seattle, WA, USA, pages 411-420; R. Feger et al "A 77-GHz FMCW MIMO Radar Based on an SiGe Single-Chip Transceiver", IEEE TRANSACTIONS ON MICROWAVE THEORY AND TECHNIQUES, VOL. 57, NO. 5, MAY 2009, pages 1020-1035; F. Garcia-Rial et al "Combining Commercially Available Active and Passive Sensors Into a Millimeter-Wave Imager for Concealed Weapon Detection", IEEE TRANSACTIONS ON MICROWAVE THEORY AND TECHNIQUES, VOL. 67, NO. 3, MARCH 2019, pages 1167-1183; Y. Lu et al "Study on feasibility of remote metal detection using millimeter wave radar for convenient and efficient security check", CCF Transactions on Pervasive Computing and Interaction (2021) 3, pages 284-299; G. Tzydynzhapov et al "New Real-Time Sub-Terahertz Security Body Scanner", Journal of Infrared, Millimeter, and Terahertz Waves, 41, 2020, pages 632-641; R. Z. Syeda et al "Sparse MIMO Array for Improved 3D mm-Wave Imaging Radar", Proceedings of the 17th European Radar Conference, 2021, pages 342-345.

Fig. 5 shows a workflow associated with a detailed embodiment of the apparatus, system and method, that utilizes millimetre wave imagery along with visible imagery in combination and acquired before and after a patient becomes partially occluded in order to determine a pose of the occluded patient. However, in one embodiment millimetre wave imagery can be utilized alone to determine the pose of the patient after occlusion and in another embodiment millimetre wave imagery acquired before and after occlusion of the patient can be utilized with visible imagery acquired prior to occlusion to determine the pose of the patient after occlusion.

Continuing with the detailed embodiment shown in Fig. 5, a RGB visible camera, for example mounted on the ceiling of the room within which a medical image acquisition unit is located acquires high resolution visible imagery of a patient on a table being prepared to be placed within the medical image acquisition unit for an image scan. A millimetre wave sensor system is also located on the ceiling of the room, and can have more than one sensor to provide for increased resolution or can have moving sensor(s) that also provides for increased resolution. However, only one millimetre wave sensor at a fixed location can be utilized. The millimetre wave sensor can for example be a multi-antenna array, employed to obtain a high spatial resolution millimetre wave image, and can be a compact single chip design operating in the 50-80 GHz band. This type of sensor captures the reflection signals of the scene and can provide 3D spatial signals. Importantly, the millimetre wave frequencies penetrate low-dielectric optical materials such as wood, plastics, and textile/fabrics. The millimetre wave sensor acquires a millimetre wave image of the patient at the same time as the RGB camera acquires a visible image of the patient.

For each frame or image pair obtained from the two cameras, dedicated processing is applied, as shown in Fig. 2. As a first step, a pose detection neural network is used to localize anatomical landmarks of the patient. If the patient is not occluded, all landmarks of the patient are detectable by the network (top row in Fig. 2). The acquired mm-wave data for this timepoint is stored along with the detected landmarks to be used for later frames.

Once an occlusion is placed onto the patient (blanket in Fig. 2, bottom row), the neural network is unable to detect all anatomical landmarks. In this case, the mm-wave data of the current frame is compared to the stored mm-wave data of the available un-occluded frames. If the mm-wave data is similar (up to a pre-defined threshold), patient motion between the two frames can be ruled out, and the stored coordinates of the occluded landmarks are transferred to the current frame. If the mm-wave data between the two frames shows marked deviations, the operator is informed that patient motion has occurred after placement of the occlusion. It is to be noted that a suitable threshold for millimetre-wave data similarity can be easily calibrated in a small volunteer study with instructed motion profiles, allowing to observe typical millimetre-wave data variations for the motion and no-motion cases.

Thus, a pose detection neural network is used to localize anatomical landmarks in the RGB data while patient is not occluded. Both these landmarks as well as the corresponding millimetre wave sensor data is stored. Once an occlusion is placed on the patient, the current mm-wave sensor is compared to the stored data from the un-occluded frame. If no change is detected, the stored landmarks are transferred to the current frame.

The pose detection neural network can be trained on a series of image pairs of a patient or patients having no occlusion and various degrees of occlusion and with no movement between image pairs and also with movement between image pairs to enable the neural network to analyse a RGB/millimetre wave image pair for a non-occluded case and then analyse a RGB/millimetre wave image pair for an occluded case, and where the pose of the patient can be determined even if the patient has moved. Thus, if the patient has moved but the movement will not cause problems for the subsequent medical image scan the operator need not be informed with respect to returning the patient to the original position but can be informed that the patient has moved in which case the operator can request the patient to stop moving or simply remove the occlusion temporarily.

The millimetre wave image data as well as being used in determining the pose of the patient can also be used for detection of metal objects on the patient support. Due to the ability to penetrate clothes and other fabrics, even hidden objects such as bracelets, piercings, etc. can be detected. A neural network can be used to aid metal object detection, but is not required. Localization of detected metal objects can then be visualized on the RGB image to provide guidance to the operator, who can then remove the objects. This can be particularly relevant for MRI scans, where ferromagnetic objects are a serious safety concern.

Rather than use an RGB camera an infrared or depth (time-of-flight) sensor can be utilized, along with a neural network that is trained to perform pose detection on this particular image in conjunction with the millimetre wave image data.

A millimetre wave source can be added to the system to illuminate the scene (active imaging), thereby enabling higher SNR in the received data. However, passive millimetre wave imaging can be utilized where no source of millimetre wave radiation is required.

In another exemplary embodiment, a computer program or computer program element is provided that is characterized by being configured to execute the method steps of any of the methods according to one of the preceding embodiments, on an appropriate apparatus or system.

The computer program element might therefore be stored on a computer unit, which might also be part of an embodiment. This computing unit may be configured to perform or induce performing of the steps of the method described above. Moreover, it may be configured to operate the components of the above-described system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A patient pose detection apparatus (10), comprising:
- at least one millimetre wave and/or terahertz sensor (20); and
- a processing unit (30);
wherein the at least one millimetre wave and/or terahertz sensor is configured to be located in the vicinity of a patient on a patient support of a medical imaging unit;
wherein the at least one millimetre wave and/or terahertz sensor is configured to acquire millimetre wave and/or terahertz image data of the patient on the patient support of the medical imaging unit prior to medical image data of the patient being acquired by the medical imaging unit;
wherein the at least one millimetre wave and/or terahertz sensor is configured to provide the millimetre wave and/or terahertz image data of the patient on the patient support of the medical imaging unit to the processing unit; and
wherein the processing unit is configured to determine a pose of the patient on the patient support of the medical imaging unit, wherein the determination of the pose of the patient on the patient support comprises utilization of the millimetre wave and/or terahertz image data of the patient on the patient support of the medical imaging unit.

2. Apparatus according to claim 1, wherein the at least one millimetre wave and/or terahertz sensor is configured to move to a plurality of positions to acquire the millimetre wave image data of the patient on the patient support of the medical imaging unit.

3. Apparatus according to any of claims 1-2, wherein the at least one millimetre wave and/or terahertz sensor comprises a plurality of millimetre wave and/or terahertz sensors spaced apart from one another.

4. Apparatus according to any of claims 1-3, wherein the apparatus comprises a visible or infrared sensor, wherein the visible or infrared sensor is configured to be located in the vicinity of the patient on the patient support of the medical imaging unit, wherein the visible or infrared sensor is configured to acquire visible or infrared image data of the patient on the patient support of the medical imaging unit prior to medical image data of the patient being acquired by the medical imaging unit, wherein the visible or infrared sensor is configured to provide the visible or infrared image data of the patient on the patient support of the medical imaging unit to the processing unit, and wherein the determination of the pose of the patient on the patient support comprises utilization of the visible or infrared image data of the patient on the patient support of the medical imaging unit.

5. Apparatus according to claim 4, wherein the millimetre wave and/or terahertz image data of the patient on the patient support of the medical imaging unit comprises a first millimetre wave and/or terahertz image of the patient on the patient support of the medical imaging unit acquired at a first time frame and a second millimetre wave and/or terahertz image of the patient on the patient support of the medical imaging unit acquired at a second time frame after the first time frame, and wherein the visible or infrared image data of the patient on the patient support of the medical imaging unit comprises a first visible or infrared image of the patient on the patient support of the medical imaging unit acquired contemporaneously with the first millimetre wave and/or terahertz image of the patient on the patient support of the medical imaging unit.

6. Apparatus according to claim 5, wherein the processing unit is configured to determine locations of a plurality of landmarks of the patient at the first time frame, wherein the determination of the locations of the plurality of landmarks of the patient at the first time frame comprises utilization of the first millimetre wave and/or terahertz image of the patient on the patient support of the medical imaging unit and the first visible or infrared image of the patient on the patient support of the medical imaging unit, and the processing unit is configured to determine locations of the plurality of landmarks of the patient at the second time frame, wherein the determination of the locations of the plurality of landmarks of the patient at the second time frame comprises utilization of the determined locations of the plurality of landmarks of the patient at the first time frame and the second millimetre and/or terahertz wave image of the patient on the patient support of the medical imaging unit, and wherein the determination of the pose of the patient on the patient support comprises utilization of the determined locations of the plurality of landmarks of the patient at the second time frame.

7. Apparatus according to any of claims 5-6, wherein the visible or infrared image data of the patient on the patient support of the medical imaging unit comprises a second visible or infrared image of the patient on the patient support of the medical imaging unit acquired contemporaneously with the second millimetre wave and/or terahertz image of the patient on the patient support of the medical imaging unit.

8. Apparatus according to claim 7 when dependent upon claim 6, wherein the determination of the locations of the plurality of landmarks of the patient at the second time frame comprises utilization of the second visible or infrared image of the patient on the patient support of the medical imaging unit.

9. Apparatus according to any of claims 7-8, wherein the determination of the pose of the patient on the patient support comprises utilization of a trained neural network.

10. Apparatus according to claim 9, wherein the trained neural network has been trained on the basis of a plurality of image pairs of one or more reference persons, wherein the plurality of image pairs comprises millimetre wave and/or terahertz images of the one or more reference persons and associated visible or infrared images of the one or more persons acquired contemporaneously with the millimetre wave and/or terahertz images of the one or more reference persons.

11. Apparatus according to claim 10, wherein the plurality of image pairs of one or more reference persons comprises image data of the one or more reference persons with different degrees of body occlusion with respect to visible or infrared image data.

12. A patient pose detection system (100), comprising:
- a medical imaging unit (110);
- at least one millimetre wave and/or terahertz sensor (20); and
- a processing unit (30);
wherein the at least one-millimetre wave and/or terahertz sensor is located in the vicinity of a patient on a patient support of the medical imaging unit;
wherein the at least one millimetre wave and/or terahertz sensor is configured to acquire millimetre wave and/or terahertz image data of the patient on the patient support of the medical imaging unit prior to medical image data of the patient being acquired by the medical imaging unit;
wherein the at least one millimetre wave and/or terahertz sensor is configured to provide the millimetre wave and/or terahertz image data of the patient on the patient support of the medical imaging unit to the processing unit; and
wherein the processing unit is configured to determine a pose of the patient on the patient support of the medical imaging unit, wherein the determination of the pose of the patient on the patient support comprises utilization of the millimetre wave and/or terahertz image data of the patient on the patient support of the medical imaging unit.

13. A patient pose detection method (200), comprising:
acquiring (210) by at least one millimetre wave and/or terahertz sensor located in the vicinity of a patient on a patient support of a medical imaging unit millimetre wave and/or terahertz image data of the patient on the patient support of the medical imaging unit prior to medical image data of the patient being acquired by the medical imaging unit;
providing (220) by the at least one millimetre wave and/or terahertz sensor the millimetre wave and/or terahertz image data of the patient on the patient support of the medical imaging unit to a processing unit; and
determining (230) by the processing unit a pose of the patient on the patient support of the medical imaging unit, wherein the determining the pose of the patient on the patient support comprises utilizing the millimetre wave and/or terahertz image data of the patient on the patient support of the medical imaging unit.

14. A computer program element for controlling an apparatus according to any of claims 1-11 which when executed by a processor is configured to carry out the method of claim 13 or for controlling a system according to claim 12 which when executed by a processor is configured to carry out the method of claim 13.

15. A computer readable medium having stored the computer program element according to claim 14.
